# EUROPEAN PATENT APPLICATION

(11) **EP 1 566 638 A1**
(43) Date of publication of application: **24.08.2005**
(21) Application number: 03774208.7
(22) Date of filing: 25.11.2003
(51) Int. Cl.: G01N 33/543, G01N 33/553, G01N 37/00

(54) **METHOD OF FIXING BIOMOLECULE ON METAL SUPPORT**

(30) Priority: 25.11.2002 JP 2002340464
(71) Applicant: Nisshinbo Industries, Inc., Tokyo 103-8650 (JP)
(72) Inventor: Kimura, Naoki, Nisshinbo Industries, Inc., Chiba-shi, Chiba 267-0056 (JP); Oda, Ryuichi, Nisshinbo Industries, Inc., Chiba-shi, Chiba 267-0056 (JP)
(74) Representative: Bannerman, David Gardner
(86) International application number: PCT/JP2003/015010
(87) International publication number: WO 2004/048973

(57) **Abstract**

A solution of a nucleic acid is spotted on a metal carrier consisting of a metal selected from Groups I, II, III, IV, V, VI, and VII of second to seventh periods and transition elements in a periodic table, or of an alloy containing the metal, and the solution is dried. Then, the nucleic acid is immobilized on the carrier spotted with the solution by irradiating the carrier with an ultraviolet ray containing a component having a wavelength of 280 nm, of which irradiation dose is 100 mJ/cm² or more.

SEQUENCE LISTING

<110> Nisshinbo Industries, Inc.

<120> Method for immobilizing biomolecules on metalic substrate

<130> F22240P1657

<150> JP 2002-340464
<151> 2002-11-25

<160> 11

<170> PatentIn version 3.0

<210> 1
<211> 21
<212> DNA
<213> Artificial sequence

<220>
<223> Description of Artificial Sequence: capture
      oligonucleotide

<400> 1
aaatgggtac tgtgcctgtt a                                                21

<210> 2
<211> 21
<212> DNA
<213> Artificial sequence

<220>
<223> Description of Artificial Sequence: capture
      oligonucleotide

<400> 2
atgactaccg gcgcgacgat g                                                21

<210> 3
<211> 21
<212> DNA
<213> Artificial sequence

<220>
<223> Description of Artificial Sequence: probe DNA

<400> 3
tcgcccgctg tttttgatga ggcggatttt ccggcagttg ccgtttatct caccggcgct 60
gaatacacgg gcgaagagct ggacagcgat acctggcagg cggagctgca tatcgaagtt 120
ttcctgcctg ctcaggtgcc ggattcagag ctggatgcgt ggatggagtc ccggatttat 180
ccggtgatga gcgatatccc ggcactgtca gatttgatca ccagtatggt ggccagcggc 240
tatgactacc ggcgcgacga tg 262

<210> 4
<211> 31
<212> DNA
<213> Artificial sequence

<220>
<223> Description of Artificial Sequence: capture
      oligonucleotide

<400> 4
tttttttttt aaatgggtac tgtgcctgtt a 31

<210> 5
<211> 31
<212> DNA
<213> Artificial sequence

<220>
<223> Description of Artificial Sequence: capture
      oligonucleotide

<400> 5
tttttttttt atgactaccg gcgcgacgat g 31

<210> 6
<211> 31
<212> DNA
<213> Artificial sequence

<220>
<223> Description of Artificial Sequence: capture
      oligonucleotide

<400> 6
tttttttttt atgactacca gcgcgacgat g 31

<210> 7
<211> 21
<212> DNA
<213> Artificial sequence

<220>
<223> Description of Artificial Sequence: capture
oligonucleotide

<400> 7
tcgccccgct gtttttgatg a 21

<210> 8
<211> 21
<212> DNA
<213> Artificial sequence

<220>
<223> Description of Artificial Sequence: capture
oligonucleotide

<400> 8
catcgtcgcg ccggtagtca t 21

<210> 9
<211> 26
<212> DNA
<213> Artificial sequence

<220>
<223> Description of Artificial Sequence: capture
oligonucleotide

<400> 9
tttttaaatg ggtactgtgc ctgtta 26

<210> 10
<211> 26
<212> DNA

<213>  Artificial sequence

<220>
<223>  Description of Artificial Sequence: capture
       oligonucleotide

<400>  10
tttttatgac taccggcgcg acgatg                                          26

<210>  11
<211>  26
<212>  DNA
<213>  Artificial sequence

<220>
<223>  Description of Artificial Sequence: capture
       oligonucleotide

<400>  11
tttttatgac taccagcgcg acgatg                                          26

## Description

### Technical Field

The present invention relates to a method of immobilizing a biomolecule such as a nucleic acid on a carrier. The method of the present invention is useful for operations of analysis of nucleic acids based on hybridization and so forth.

### Background Art

In analyses of nucleic acids based on hybridization, immunoassays and so forth, techniques of immobilizing nucleic acids or proteins on carriers such as membranes and plates have conventionally been utilized. As such methods of immobilizing biomolecules, the following methods are known for nucleic acids:
(1) A method of chemically binding a nucleic acid introduced with a modification group, such as immobilization by a disulfide bond between a nucleic acid having a thiol group at the 5' end and a bead-like base material having thiol groups (P.J.R. Day, P.S. Flora, J.E. Fox, M.R. Walker, Biochem. J., 278, 735-740 (1991));
(2) A method of immobilizing a nucleic acid by adsorption on a carrier such as nitrocellulose, nylon membrane, or glass coated with a cation polymer such as poly-L-Lysine through ultraviolet (UV) irradiation or heat treatment (J. Sambrook, E.F. Fritsch and T. Maniatis, Molecular Cloning, Cold Spring Harbor Laboratory Press, Second Edition, pages 2.109-2.113 and pages 9.34-9.46, JP 10-503841 A);
(3) A method of immobilizing nucleic acid on the basis of physical adsorption obtained by injecting the nucleic acid into wells of a microplate treated with a polylysine solution and heating the plate at 37°C (G.C.N. Parry and A.D.B. Malcolm, Biochem. Soc. Trans., 17, 230-231 (1989));
(4) A method comprising synthesizing DNA on a base material by using nucleotides which bonded to the base material (International Publication Pamphlet No.97/10365 (WO97/10365)); and
(5) A method of immobilizing a nucleic acid on a base material such as glass carrying a polymer compound having carbodiimide groups (JP 8-23975 A).

However, the method of (1) requires an extremely special apparatus and regents. Furthermore, in the methods of (2) and (3), nucleic acids are dropped off from the carriers during the hybridization, in particular, in operation processes, and thus detection sensitivity may be reduced, or reproducibility cannot be obtained. Furthermore, those methods suffer from further drawback, that is, although a long nucleic acid can be immobilized, a short nucleic acid of about 50-mer or shorter such as oligomers cannot be efficiently immobilized. In those methods, the UV dose is about several tens mJ/cm². Furthermore, the method of (4) also requires an extremely special apparatus and regents for synthesizing DNA on a base material, and the nucleic acid that can be synthesized is limited to about 25-mer or shorter. Moreover, in the method of (5), the material of the base material is limited, and a surface coating step is required.

### Disclosure of the Invention

In view of the aforementioned technical situations of the conventional techniques, an object of the present invention is to provide a method of conveniently and efficiently immobilizing a biomolecule such as a nucleic acid, in particular, a nucleic acid of a short chain length, on a carrier.

The inventors of the present invention have conducted various researches in order to achieve the aforementioned object. As a result, they have found that a nucleic acid can be efficiently immobilized on a carrier by spotting a solution of the nucleic acid on a metallic carrier and then irradiating the carrier with an ultraviolet ray, and have accomplished the present invention.

Thus, the present invention provides the following.
(1) A method of immobilizing a biomolecule on a carrier, including the steps of: spotting a solution of the biomolecule on the carrier; and irradiating the carrier spotted with the solution of the biomolecule with an ultraviolet ray containing a component having a wavelength of 280 nm, in which the carrier is made of a metal.
(2) The method according to (1), in which the ultraviolet ray contains a component having a wavelength of 220 to 300 nm.
(3) The method according to (1) or (2), in which the metal is a metal selected from Groups I, II, III, IV, V, VI, and VII of second to seventh periods and transition elements in a periodic table, or an alloy containing any of these metal.
(4) The method according to any one of (1) to (3), in which irradiation dose of the ultraviolet ray is 100 mJ/cm² or more.
(5) The method according to any one of (1) to (4), in which the biomolecule is selected from a nucleic acid, protein, saccharide, antigen, antibody, peptide, and enzyme.
(6) A method of producing a biomolecule-immobilized carrier in which a biomolecule is immobilized on a carrier, including the steps of: spotting a solution of the biomolecule on the carrier; and irradiating the carrier spotted with the solution of the biomolecule with an ultraviolet ray containing a component having a wavelength of 280 nm to immobilize the biomolecule on the carrier.
(7) The method according to (6), in which the ultraviolet ray contains a component having a wavelength of 220 to 300 nm.
(8) The method according to (6) or (7), in which the biomolecule is a nucleic acid, and the nucleic acid-immobilized carrier is used for analysis of the nucleic acid by hybridization.

### Brief Description of the Drawing

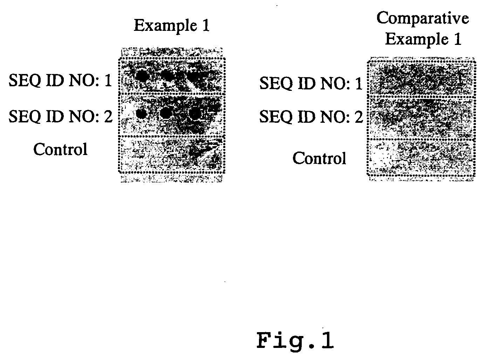

Fig. 1 (photograph) shows the result of hybridization using the oligonucleotide-immobilized plate produced in the example.

The dotted line represents the regions on which the oligonucleotides were immobilized, and the regions on which 1 × TE buffer solution was spotted as a control in Example 1 and Comparative Example 1.

### Best Mode for carrying out the Invention

Hereafter, the present invention will be explained in detail.

A carrier used in the present invention is intended to immobilize a biomolecule, and is made of a metal. The metal is not particularly limited as long as it can immobilize a biomolecule by ultraviolet ray irradiation. Preferable examples thereof include a metal selected from Groups I, II, III, IV, V, VI, and VII of second to seventh periods and transition elements in a periodic table, and an alloy containing each of these metal.

Particularly preferable examples of the metal selected from Groups I, II, III, IV, V, VI, and VII of second to seventh periods and transition elements in the periodic table include aluminum, titanium, platinum, tungsten, molybdenum, gold, copper, and nickel.

Specific examples of the alloy include nickel silver (component: Cu, Ni, Zn), brass (component: Cu, Zn), bronze (component: Cu, Be), Monel (component: Cu, Ni, Fe, Mn), a nickel-cobalt alloy (component: Ni, Co), a nickel-chromium alloy (component: Ni, Cr), a cobalt alloy (component: Co, Ni, Cr), stainless steel (component: Ni, Cr, Fe), silver tungsten (component: Ag, W), β titanium (component: Ti, V, Al),αβ titanium (component: Ti, V, Al), an NT alloy (component: Ti, Ni), an aluminum alloy (component: Al, Cu, Mg, Si, Mn, Zn), duralumin (component: Al, Cu, Si, Fe, Mn, Mg, Zn), a magnesium alloy (component: Mg, Al, Zn), K24 (component: Au), K18 (component: Au, Ag, Cu), beryllium copper (component: Cu, Be), cast iron (component: Fe, Mn, S, C), carbon steel (component: Fe, C, Si, Mn, P, S), bronze casting (component: Cu, Sn, Zn, Pb), phosphor bronze casting (component: Cu, Zn, P), brass casting (component: Cu, Zn, Pb), manganese brass (component: Cu, Zn, Mn, Fe, Al), silzin bronze casting (component: Cu, Si, Zn), aluminum bronze casting (component: Cu, Al, Fe, Ni, Mn), elinvar (component: Ni, Cr, Mn), elinvar extra (component: Ni, Cr, Co, Mn), invar (component: Ni, Fe), super invar (component: Fe, Ni, Co), stainless invar (component: Fe, Co, Cr), Malottes (component: Sn, Bi, Pb), Lipowitz (component: Sn, Bi, Pb, Cd), Wood's (component: Sn, Bi, Pb, Cd), manganin (component: Cu, Mn, Ni, Fe), izabellin (component: Cu, Mn, Al), constantan (component: Cu, Ni), arcless (component: Fe, Cr, Al), kanthal (component: Cr, Fe, Al, Co), alumel (component: Ni, Al), a magnetic material (a material containing a ferromagnetic transition element such as Fe, Ni, or Co), permalloy (component: Fe, Ni), alpalm (component: Fe, Al), ferrite (complex oxide having Fe₂O₃ as a main component), sendust (component: Fe, Si, Al), super sendust (component: Fe, Si, Al, Ni), Alnico (component: Fe, Al, Ni, Co), a hydrogen absorbing metal (such as a lanthanum nickel alloy (component: La, Ni)), a Co-Cr based alloy, a SnO₂ based oxide, an Nb-Ti alloy, a damping alloy (such as an alloy material which reduces and absorbs vibration, and blocks propagation of vibration, for example, an Al-Zn super plastic alloy, a silent alloy, or nitinol), a material for an electrode, and a semiconductor material (such as silicon, germanium, or potassium arsenide).

The metal may also be deposited or plated with another metal. Further the metal may have different kinds of metals laminated thereon, or may be a single metal to retain its shape.

A carrier according to the present invention consists essentially of the metal. The carrier may consist of the metal only, or may have the metal laminated on a non-metal material by adhesion, deposition, plating, or the like.

The shape of the aforementioned carrier is not particularly limited, and examples of the shape include those of foil, plate, wafer, filter, and bead. Furthermore, it may be the shape of microtiter plate. In order to facilitate preservation of the obtained result, a back surface of plate or the like may be, for example, applied or coated with a material usable as a seal or the like (adhesives etc.) so that the material can be used as a seal.

A solution of a biomolecule is spotted on predetermined positions of the aforementioned carrier. Examples of the biomolecule include nucleic acids, proteins, saccharides, antigens, antibodies, peptides, and enzymes. Hereafter, explanation will be made by exemplifying a nucleic acid as the biomolecule. However, methods and conditions conventionally used for immobilization can be used also for other substances except that an ultraviolet ray is applied for the immobilization.

The nucleic acid is not particularly different from a usual solid phase-immobilized nucleic acid used for hybridization of nucleic acids using a solid phase-immobilized nucleic acid, and it is not particularly limited so long as it is a nucleic acid that allows hybridization. For example, it may be a naturally occurring or synthesized DNA (including oligonucleotides) or RNA (including oligonucleotides). Further, the nucleic acid may be single-stranded or double-stranded. The chain length of the nucleic acid is not also particularly limited so long as it allows hybridization. However, it is usually about 5 to 50,000 nucleotides, preferably 20 to 10,000 nucleotides. Furthermore, the nucleic acid may have a polymer of oligonucleotides having a group that becomes reactive upon ultraviolet ray irradiation such as those of thymidine or the like at the 5' end or 3' end.

The solvent for dissolving the nucleic acid is not particularly limited, and examples thereof include: distilled water; buffers usually used for preparing a nucleic acid solution, for example, a Tris buffer such as TE buffer (10 mM Tris/hydrochloric acid, pH 8.0, 1 mM EDTA); an aqueous solution containing sodium chloride; an aqueous solution containing a carboxylic acid salt (sodium citrate, ammonium citrate, sodium acetate etc.); an aqueous solution containing a sulfonic acid salt (sodium dodecylsulfate, ammonium dodecylsulfate etc.); and an aqueous solution containing a phosphonic acid salt (sodium phosphate, ammonium phosphate etc.). Examples thereof further include commercially available solvents such as Micro Spotting Solution (TeleCHem International, Inc.). Although the concentration of the nucleic acid solution is not particularly limited either, it is usually a concentration of 1 mmol/ml to 1 fmol/ml, preferably 100 pmol/ml to 100 fmol/ml.

Examples of the method of spotting of the nucleic acid solution on the carrier include a method involving dropping the nucleic acid solution onto the carrier with a pipette and a method involving using a commercially available spotter. Although the shape of spot and amount of the solution to be spotted are not particularly limited so long as the position at which the nucleic acid solution has been spotted can be confirmed, the shape is preferably a dot shape or circular shape. The amount of the solution to be spotted is preferably 10 nl to 10 ml. The nucleic acid solution is spotted on one place or two or more places on the carrier. One kind of nucleic acid solution or two or more kinds of nucleic acid solutions may be spotted. A labeled nucleic acid may be immobilized as a positive control indicating immobilization of the nucleic acid on the carrier.

In a preferred embodiment of the present invention, after the nucleic acid solution is spotted on the carrier, an ultraviolet ray containing a component having a wavelength of 280 nm is applied. An ultraviolet ray containing a component having a wavelength of 220 to 300 nm can be given as the ultraviolet ray. The nucleic acid solution can be dried after the spotting and before the ultraviolet ray irradiation. The nucleic acid solution may be spontaneously dried, or dried by heating. When it is heated, the heating temperature is usually 30 to 100°C, and preferably 35 to 45°C.

Then, an ultraviolet ray containing a component having a wavelength of 280 nm is applied at least on the position or positions of the carrier at which the nucleic acid has been immobilized. Specifically, the ultraviolet ray may be monochromatic light having a wavelength of 280 nm, or an ultraviolet ray having a broad waveform and containing a component having a wavelength of 280 nm. Examples of the ultraviolet ray having a broad waveform and containing a component having a wavelength of 280 nm include an ultraviolet ray containing a component having a wavelength of 220 to 300 nm. Examples of the ultraviolet ray containing a component having a wavelength of 220 to 300 nm include an ultraviolet ray having a maximum value at near 280 nm. The irradiation dose is usually 100 mJ/cm² or more, preferably 200 mJ/cm² or more, as cumulative irradiation dose.

By immobilizing a nucleic acid on a carrier as described above, a nucleic acid-immobilized carrier is produced. The nucleic acid-immobilized carrier obtained by the method of the present invention can be used for, for example, analysis of nucleic acids based on hybridization. Because a nucleic acid immobilized on a carrier by the method of the present invention hardly detaches from the carrier under the conditions of usual hybridization, more favorable detection sensitivity and reproducibility can be obtained than those in the case where the irradiation with an ultraviolet ray is not performed. The hybridization and detection thereof can be performed in the same manner as hybridization utilizing a usual solid phase-immobilized nucleic acid.

Since an inexpensive metallic material is used as a carrier for immobilizing a nucleic acid in the present invention, the cost can be reduced. Moreover, since the metallic material can be easily formed, it becomes easy to produce DNA microarrays of various shapes. Further, the metallic material can be stored for a long period of time, and has superior storage stability. Furthermore, the method of the present invention does not require a step of coating a surface of carrier, and thus it becomes possible to immobilize nucleic acid directly on a metal used as an electrode or the like. Hybridization of a complementary nucleic acid in the solution and an immobilized nucleic acid can be performed efficiently by immobilizing a nucleic acid on an electrode. Hybridization is presumably performed efficiently because a nucleic acid, which has negative charge, is attracted to a positive electrode and then the concentration of the nucleic acid near the positive electrode becomes high.

### Examples

Hereafter, the present invention will be explained more specifically by way of examples.

### Example 1: Immobilization of nucleic acid on plate

Oligonucleotides having the nucleotide sequences of SEQ ID NOS: 1 and 2 respectively (21mer) were synthesized in a conventional manner by using an oligonucleotide synthesizer (Perkin-elmer Applied Biosystems). Furthermore, DNA having the nucleotide sequence of SEQ ID NO: 3 (262bp) was also prepared as a probe. The oligonucleotide having the nucleotide sequence of SEQ ID NO: 1 and the probe were biotinylated at the 5' ends. The oligonucleotide having the nucleotide sequence of SEQ ID NO: 2 was complementary to the biotinylated probe. Those oligonucleotides were dissolved in 1 × TE buffer (10 mM Tris-HCl, pH 8/ 1 mM EDTA) at a concentration of 1 pmol/µl.

Each of the aforementioned oligonucleotide solutions was spotted on a commercially available aluminum foil (Mitsubishi Aluminum Co., Ltd.) as three spots at predetermined positions (Fig. 1). The amount of the solutions used for each spotting was 0.5 µl, and the size of each of the spots was about 1 mm in diameter. This aluminum foil was put into a drier and dried at 37°C for 20 minutes. Then, the foil was irradiated with an ultraviolet ray containing a component having a wavelength of 280 nm for 250 mJ/cm² by using Uvstratalinker 2400 (STRATAGENE) at a distance of 16 cm. The irradiation time was 100 seconds. Then, the aluminum foil was washed by shaking in water for 30 minutes, and dried.

On the other hand, a solution not containing any nucleic acid (1 × TE buffer) was also subjected to the immobilization operation as a control by spotting it on the aluminum foil in a similar manner.

### Comparative Example 1

The aluminum foil was irradiated beforehand with an ultraviolet ray containing a component having a wavelength of 280 nm for 250 mJ/cm² by using Uvstratalinker 2400 (STRATAGENE) at a distance of 16 cm. Each of the oligonucleotide solutions described in Example 1 was spotted on the aluminum foil as three spots at predetermined positions. The amount of the solutions used for each spotting was 0.5 µl, and the size of each of the spots was about 1 mm in diameter. The irradiation time was 100 seconds. This aluminum foil was put into a drier and dried at 37°C for 20 minutes. Then, the aluminum foil was washed by shaking in water for 30 minutes, and dried.

On the other hand, a solution not containing any nucleic acid (1 × TE buffer) was also subjected to the immobilization operation as a control by spotting it on the aluminum foil in a similar manner.

### Example 2: Hybridization and detection thereof

### (1) Hybridization

On the nucleic acid-immobilized portions of the oligonucleotide-immobilized aluminum foil of Example 1 and Comparative Example 1, 60 µl of a hybridization solution (Arrayit UniHyb, (TeleCHem International, Inc.) containing 3 pmol of the biotinylated probe (262bp) was placed, and the aluminum foil was put into a case shielded from water (HybriCassette), immersed in a water bath with the case, and heated at 45°C for 2 hours.

### (2) Post-hybridization

After the hybridization, post-hybridization washing was performed under the following conditions to remove the probe non-specifically adsorbed on the oligonucleotide-immobilized aluminum foil.

### [Post-hybridization washing conditions]

1) 2 × SSC, 0.1% SDS; room temperature, 5 minutes, twice
2) 0.2 × SSC, 0.1% SDS; 40°C, 5 minutes, twice
3) 2 × SSC; room temperature, 1 minute, 3 times

### (3) Detection of oligonucleotides immobilized on aluminum foil and hybridization

On the portions of the aluminum foil on which the hybridization solution was placed, 1.5 ml of a blocking solution containing milk proteins (BlockAce, Snow Brand Milk Products) was placed to perform blocking at room temperature for 30 minutes. After the blocking solution was removed, 1.5 ml of streptavidin-alkaline phosphatase conjugate solution (VECTOR) was placed and reacted at room temperature for 30 minutes. Then, the aluminum foil was immersed in TBST solution (50 mM Tris-HCl (pH 7.5), 0.15 M NaCl, 0.05% Tween 20) and shaken for 5 minutes to remove the conjugate that did not react. Finally, 1.5 ml of a substrate solution (TMB) was placed on the portions of the aluminum foil on which the hybridization solution was placed and left for 30 minutes to perform a coloring reaction.

The results are shown in Table 1. The symbols used in Table 1 have the same meanings as in Table 2 and the other tables mentioned later. The signals of the positions at which the oligonucleotide having the sequence of SEQ ID NO: 1 was immobilized indicate amounts of immobilized oligonucleotides, and the signals of the positions at which the oligonucleotide having the sequence of SEQ ID NO: 2 was immobilized indicate intensities of hybridization.

**Table 1**

| | Immobilized oligonucleotide | |
|---|---|---|
| | SEQ ID NO: 1 | SEQ ID NO: 2 |
| Example 1 | ⓞ | ⓞ |
| Comparative Example 1 | × | × |
| ⓞ: Most of signals appeared extremely clearly with extremely high sensitivity. | | |
| ○ : Most of signals appeared clearly with high sensitivity. | | |
| Δ: A part of signals appeared unclearly or with low sensitivity. | | |
| ×: Most of signals appeared unclearly or with low sensitivity, or no signal appeared at all. | | |

As apparent from the results shown in Table 1, the oligonucleotides were more surely immobilized on the oligonucleotide-immobilized aluminum foil of Example 1 than on the oligonucleotide-immobilized aluminum foil of Comparative Example 1. Moreover, on the oligonucleotide-immobilized aluminum foil of Example 1, the hybridization signals appeared clearly. In addition, on the positions of control (positions at which a solution not containing any nucleic acid was spotted), no signal appeared at all.

### Example 3: Immobilization of nucleic acid on plate

Oligonucleotides having the nucleotide sequences of SEQ ID NOS: 4, 5, and 6 respectively (31mer) were synthesized in a conventional manner by using an oligonucleotide synthesizer (Perkin-elmer Applied Biosystems). The oligonucleotide having the nucleotide sequence of SEQ ID NO: 4 was biotinylated at the 5' end. The oligonucleotides having the nucleotide sequences of SEQ ID NOS: 4 and 5 corresponded to the oligonucleotides having the nucleotide sequences of SEQ ID NOS: 1 and 2 described in Example 1 with ten thymidine residues at the 5' ends, respectively. The oligonucleotide of SEQ ID NO: 5 was complementary to the aforementioned biotinylated probe, and the oligonucleotide of SEQ ID NO: 6 did not have complementarity because it was different from the oligonucleotide of SEQ ID NO: 5 by one nucleotide. Those oligonucleotides were dissolved in 5 × SSC at a concentration of 100 pmol/ml.

Each of the aforementioned oligonucleotide solutions was spotted on a commercially available stainless plate (Special Kinzoku Kogyo Co., Ltd.) as three spots at predetermined positions by using a spotter (Pyxsis 5500, CARTESIAN). The size of each of the spots was about 0.3 mm in diameter. This plate was put into a drier and dried at 42°C for 20 minutes. Then, the plate was irradiated with an ultraviolet ray containing a component having a wavelength of 280 nm for 300 mJ/cm² by using Uvstratalinker 2400 (STRATAGENE) at a distance of 16 cm. The irradiation time was 120 seconds. Then, the plate was washed by shaking in water for 30 minutes, and dried.

On the other hand, a solution not containing any nucleic acid (2 × SSC buffer) was also subjected to the immobilization operation as a control by spotting it on the plate in a similar manner.

### Comparative Example 2

The stainless plate was irradiated beforehand with an ultraviolet ray containing a component having a wavelength of 280 nm for 300 mJ/cm² by using Uvstratalinker 2400 (STRATAGENE) at a distance of 16 cm. Each of the oligonucleotide solutions described in Example 3 was spotted on the stainless plate as three spots at predetermined positions by using a spotter (Pyxsis 5500, CARTESIAN). The irradiation time was 120 seconds. This plate was put into a drier and dried at 42°C for 20 minutes. Then, the plate was washed by shaking in water for 30 minutes, and dried.

On the other hand, a solution not containing any nucleic acid (2 × SSC buffer) was also subjected to the immobilization operation as a control by spotting it on the plate in a similar manner.

### Example 4: Hybridization and detection thereof

On the nucleic acid-immobilized portions of the oligonucleotide-immobilized plates of Example 3 and Comparative Example 2, 60 ml of a hybridization solution (Arrayit UniHyb, (TeleCHem International, Inc.) containing 3 pmol of the biotinylated probe (262bp) was placed, and the plates were put into a case shielded from water (HybriCassette), immersed in a water bath with the case, and heated at 45°C for 2 hours.

Thereafter, post-hybridization, detection of the oligonucleotides immobilized on the plates, and hybridization were performed in the same manner as that of Example 2. The results are shown in Table 2. The signals of the positions at which the oligonucleotide having the sequence of SEQ ID NO: 4 was immobilized indicate amounts of immobilized oligonucleotides, and the signals of the positions at which the oligonucleotide having the sequence of SEQ ID NO: 5 was immobilized indicate intensities of hybridization.

**Table 2**

| | Immobilized oligonucleotide | | |
|---|---|---|---|
| | SEQ ID NO: 4 | SEQ ID NO: 5 | SEQ ID NO: 6 |
| Example 3 | ⓞ | ⓞ | × |
| Comparative Example 2 | × | × | × |

As apparent from the results shown in Table 2, the oligonucleotides were more surely immobilized on the oligonucleotide-immobilized plate of Example 3 than on the oligonucleotide-immobilized plate of Comparative Example 2. Moreover, on the oligonucleotide-immobilized plate of Example 3, the hybridization signals also appeared clearly. In addition, on the positions of control (positions at which a solution not containing any nucleic acid was spotted) and SEQ ID NO: 6, no signal appeared at all.

### Example 5: Immobilization of nucleic acid on plate

Each of the oligonucleotide solutions prepared in example 3 was spotted on a silver tungsten plate (Eastern Technics Corp.) as three spots at predetermined positions by using a spotter. The size of each of the spots was about 0.3 mm in diameter. This plate was put into a drier and dried at 42°C for 20 minutes. Then, the plate was irradiated with an ultraviolet ray containing a component having a wavelength of 280 nm for 400 mJ/cm² by using Uvstratalinker 2400 (STRATAGENE) at a distance of 16 cm. The irradiation time was 160 seconds. Then, the plate was washed by shaking in water for 30 minutes, and dried.

On the other hand, a solution not containing any nucleic acid (2 × SSC buffer) was also subjected to the immobilization operation as a control by spotting it on the plate in a similar manner.

### Comparative Example 3

The silver tungsten plate was irradiated beforehand with an ultraviolet ray containing a component having a wavelength of 280 nm for 400 mJ/cm² by using Uvstratalinker 2400 (STRATAGENE) at a distance of 16 cm. Each of the oligonucleotide solutions described in Example 3 was spotted on the silver tungsten plate as three spots at predetermined positions by using a spotter (Pyxsis 5500, CARTESIAN). The irradiation time was 160 seconds. This plate was put into a drier and dried at 42°C for 20 minutes. Then, the plate was washed by shaking in water for 30 minutes, and dried.

On the other hand, a solution not containing any nucleic acid (2 × SSC buffer) was also subjected to the immobilization operation as a control by spotting it on the plate in a similar manner.

### Example 6: Hybridization and detection thereof

On the nucleic acid-immobilized portions of the oligonucleotide-immobilized plates of Example 5 and Comparative Example 3, 60 ml of a hybridization solution (Arrayit UniHyb, (TeleCHem International, Inc.) containing 3 pmol of the biotinylated probe (262bp) was placed, and the plates were put into a case shielded from water (HybriCassette), immersed in a water bath with the case, and heated at 45°C for 2 hours.

Thereafter, post-hybridization, detection of the oligonucleotides immobilized on the plates, and hybridization were performed in the same manner as that of Example 2. The results are shown in Table 3.

**Table 3**

| | Immobilized oligonucleotide | | |
|---|---|---|---|
| | SEQ ID NO: 4 | SEQ ID NO: 5 | SEQ ID NO: 6 |
| Example 5 | ⓞ | ⓞ | × |
| Comparative Example 3 | × | × | × |

As apparent from the results shown in Table 3, the oligonucleotides were more surely immobilized on the oligonucleotide-immobilized plate of Example 5 than on the oligonucleotide-immobilized plate of Comparative Example 3. Moreover, on the oligonucleotide-immobilized plate of Example 5, the hybridization signals also appeared clearly. In addition, on the positions of control (positions at which a solution not containing any nucleic acid was spotted) and SEQ ID NO: 6, no signal appeared at all.

### Example 7: Immobilization of nucleic acid on plate

A λ DNA fragment (A) was amplified in a conventional manner by using oligonucleotides having the nucleotide sequences shown in SEQ ID NOS: 7 and 8 as primers. The obtained fragment was subjected to agarose electrophoresis and detected by ethidium bromide staining. As a result, it was found that the fragment had a length of about 300b. A λ DNA fragment (B) (about 300b) that was not complementary to the aforementioned λ DNA was also amplified in a similar manner.

A solution of each of the aforementioned λ DNAs was spotted on a commercially available aluminum foil (Mitsubishi Aluminum Co., Ltd.) as three spots at predetermined positions by using a spotter (Pyxsis 5500, CARTESIAN). The size of each of the spots was about 0.3 mm in diameter. This aluminum foil was put into a drier and dried at 42°C for 20 minutes. Then, the foil was irradiated with an ultraviolet ray containing a component having a wavelength of 280 nm for 600 mJ/cm² by using Uvstratalinker 2400 (STRATAGENE) at a distance of 16 cm. The irradiation time was 240 seconds. Then, the aluminum foil was washed by shaking in water for 30 minutes, and dried.

On the other hand, a solution not containing any nucleic acid (1 × TE buffer) was also subjected to the immobilization operation as a control by spotting it on the aluminum foil in a similar manner.

### Comparative Example 4

Each of the λ DNA solutions described in Example 7 (concentration: 1 pmol/µl) was spotted on the aluminum foil as three spots at predetermined positions by using a spotter (Pyxsis 5500, CARTESIAN). This aluminum foil was put into a drier and dried at 42°C for 20 minutes. Then, the aluminum foil was washed by shaking in water for 30 minutes, and dried.

On the other hand, a solution not containing any nucleic acid (1 × TE buffer) was also subjected to the immobilization operation as a control by spotting it on the aluminum foil in a similar manner.

### Example 8: Hybridization and detection thereof

### (1) Hybridization

A λ DNA fragment (C) was amplified by using an oligonucleotide having the nucleotide sequence shown in SEQ ID NO: 7 labeled with biotin at the 5' end and an oligonucleotide having the nucleotide sequence shown in SEQ ID NO: 8 as primers. The sequence of this λ DNA fragment (C) was the same as that of λ DNA fragment (A) prepared in Example 7.

The λ DNA-immobilized aluminum foil of each of Example 7 and Comparative Example 4 was immersed in water heated to 95°C for 5 minutes, and immersed in water cooled to 4°C for 5 minutes. Subsequently, on the nucleic acid-immobilized portions of the λ DNA-immobilized aluminum foil, 60 ml of a hybridization solution (Arrayit UniHyb, (TeleCHem International, Inc.) containing 0.5 pmol of the aforementioned biotinylated λ DNA fragment (C) was placed, and the aluminum foil was put into a case shielded from water (HybriCassette), immersed in a water bath with the case, and heated at 55°C for 2 hours.

### (2) Post-hybridization

After the hybridization, post-hybridization washing was performed under the following conditions to remove the probe non-specifically adsorbed on the λ DNA-immobilized aluminum foil.

### [Post-hybridization washing conditions]

1) 2 × SSC, 0.1% SDS; room temperature, 5 minutes, twice
2) 0.2 × SSC, 0.1% SDS; 40°C, 5 minutes, twice
3) 2 × SSC; room temperature, 1 minute, 3 times

### (3) Detection of hybridization

On the portions of the aluminum foil on which the hybridization solution was placed, 1.5 ml of a blocking solution containing milk proteins (BlockAce, Snow Brand Milk Products) was placed to perform blocking at room temperature for 30 minutes. After the blocking solution was removed, 1.5 ml of streptavidin-alkaline phosphatase conjugate solution (VECTOR) was placed and reacted at room temperature for 30 minutes. Then, the aluminum foil was immersed in TBST solution (50 mM Tris-HCl (pH 7.5), 0.15 M NaCl, 0.05% Tween 20) and shaken for 5 minutes to remove the conjugate that did not react. Finally, 1.5 ml of a substrate (TMB) solution was placed on the portions of the aluminum foil on which the hybridization solution was placed and left for 30 minutes to perform a coloring reaction.

The results are shown in Table 4.

**Table 4**

| | Immobilized nucleic acid | |
|---|---|---|
| | λ DNA fragment (A) | λ DNA fragment (B) |
| Example 7 | ⓞ | × |
| Comparative Example 4 | × | × |

As apparent from the results shown in Table 4, the λ DNA fragments were surely immobilized on the λ DNA-immobilized aluminum foil of Example 7, because the hybridization signals specifically and clearly appeared on the λ DNA-immobilized aluminum foil. On the other hand, on the λ DNA-immobilized aluminum foil of Comparative Example 4, no signal appeared at all. In addition, on the positions of control (positions at which a solution not containing any nucleic acid was spotted) of the λ DNA-immobilized aluminum foil of Example 7 and the λ DNA-immobilized aluminum foil of Comparative Example 4, no signal appeared at all.

### Example 9: Immobilization of nucleic acid on plate

Each of the λ DNA solutions described in Example 7 was spotted on a commercially available stainless plate (Special Kinzoku Kogyo Co., Ltd.) as three spots at predetermined positions by using a spotter (Pyxsis 5500, CARTESIAN). The size of each of the spots was about 0.3 mm in diameter. This plate was put into a drier and dried at 42°C for 20 minutes. Then, the plate was irradiated with an ultraviolet ray containing a component having a wavelength of 280 nm for 1,200 mJ/cm² by using Uvstratalinker 2400 (STRATAGENE) at a distance of 16 cm. The irradiation time was 480 seconds. Then, the plate was washed by shaking in water for 30 minutes, and dried.

On the other hand, a solution not containing any nucleic acid (1 × TE buffer) was also subjected to the immobilization operation as a control by spotting it on the plate in a similar manner.

### Comparative Example 5

Each of the λ DNA solutions described in Example 7 was spotted on the stainless plate as three spots at predetermined positions by using a spotter (Pyxsis 5500, CARTESIAN). This plate was put into a drier and dried at 42°C for 20 minutes. Then, the plate was washed by shaking in water for 30 minutes, and dried.

On the other hand, a solution not containing any nucleic acid (1 × TE buffer) was also subjected to the immobilization operation as a control by spotting it on the plate in a similar manner.

### Example 10: Hybridization and detection thereof

The λ DNA-immobilized plates of Example 9 and Comparative Example 5 were immersed in water heated to 95°C for 10 minutes, and immersed in water cooled to 4°C for 5 minutes. Subsequently, on the nucleic acid-immobilized portions of the λ DNA-immobilized plates, 60 ml of a hybridization solution (Arrayit UniHyb, (TeleCHem International, Inc.) containing 1 pmol of the biotinylated λ DNA (C) described in Example 8 was placed, and the plates were put into a case shielded from water (HybriCassette), immersed in a water bath with the case, and heated at 60°C for 2 hours.

Thereafter, post-hybridization and detection of hybridization were performed in the same manner as that of Example 8. The results are shown in Table 5.

**Table 5**

| | Immobilized nucleic acid | |
|---|---|---|
| | λ DNA fragment (A) | λ DNA fragment (B) |
| Example 9 | ⓞ | × |
| Comparative Example 5 | × | × |

As apparent from the results shown in Table 5, the λ DNA fragments were surely immobilized on the λ DNA-immobilized plate of Example 9, because the hybridization signals specifically and clearly appeared on the plate. On the other hand, no signal appeared at all on the λ DNA-immobilized plate of Comparative Example 5. In addition, also on the positions of control (positions at which a solution not containing any nucleic acid was spotted) of the λ DNA-immobilized plate of Example 9 and the λ DNA-immobilized plate of Comparative Example 5, no signal appeared at all.

### Example 11: Immobilization of nucleic acid on plate

Each of the λ DNA solutions described in Example 7 was spotted on a commercially available silicon wafer (Mitsubishi Sumitomo Silicon Corp.) as three spots at predetermined positions by using a spotter (Pyxsis 5500, CARTESIAN). The size of each of the spots was about 0.3 mm in diameter. This silicon wafer was put into a drier and dried at 42°C for 20 minutes. Then, the silicon wafer was irradiated with an ultraviolet ray containing a component having a wavelength of 280 nm for 1,200 mJ/cm² by using Uvstratalinker 2400 (STRATAGENE) at a distance of 16 cm. The irradiation time was 480 seconds. Then, the silicon wafer was washed by shaking in water for 30 minutes, and dried.

On the other hand, a solution not containing any nucleic acid (1 × TE buffer) was also subjected to the immobilization operation as a control by spotting it on the silicon wafer in a similar manner.

### Comparative Example 6

Each of the λ DNA solutions (concentration: 1 pmol/µl) described in Example 7 was spotted on the silicon wafer as three spots at predetermined positions by using a spotter (Pyxsis 5500, CARTESIAN). This silicon wafer was put into a drier and dried at 42°C for 20 minutes. Then, the silicon wafer was washed by shaking in water for 30 minutes, and dried.

### Example 12: Hybridization and detection thereof

The λ DNA-immobilized silicon wafer of each of Example 11 and Comparative Example 6 was immersed in water heated to 95°C for 10 minutes, and immersed in water cooled to 4°C for 5 minutes. Subsequently, on the nucleic acid-immobilized portions of the λ DNA-immobilized silicon wafer, 60 ml of a hybridization solution (Arrayit UniHyb, (TeleCHem International, Inc.) containing 1 pmol of the biotinylated λ DNA (C) described in Example 8 was placed, and the silicon wafer was put into a case shielded from water (HybriCassette), immersed in a water bath with the case, and heated at 60°C for 2 hours.

Thereafter, post-hybridization and detection of hybridization were performed in the same manner as that of Example 8. The results are shown in Table 6.

**Table 6**

| | Immobilized nucleic acid | |
|---|---|---|
| | λ DNA fragment (A) | λ DNA fragment (B) |
| Example 11 | ⓞ | × |
| Comparative Example 6 | × | × |

As apparent from the results shown in Table 6, the λ DNA fragments were surely immobilized on the λ DNA-immobilized silicon wafer of Example 11, because the hybridization signals specifically and clearly appeared on the silicon wafer. On the other hand, no signal appeared at all on the λ DNA-immobilized silicon wafer of Comparative Example 6. In addition, also on the positions of control (positions at which a solution not containing any nucleic acid was spotted) of the λ DNA-immobilized silicon wafer of Example 11 and the λ DNA-immobilized silicon wafer of Comparative Example 6, no signal appeared at all.

### Example 13: Immobilization of nucleic acid on plate

Each of the λ DNA solutions described in Example 7 was spotted on a substrate obtained by subjecting a glass plate to gold evaporation as three spots at predetermined positions by using a spotter (Pyxsis 5500, CARTESIAN). The size of each of the spots was about 0.3 mm in diameter. This gold-evaporated glass substrate was put into a drier and dried at 42°C for 20 minutes. Then, the substrate was irradiated with an ultraviolet ray containing a component having a wavelength of 280 nm for 1,200 mJ/cm² by using Uvstratalinker 2400 (STRATAGENE) at a distance of 16 cm. The irradiation time was 480 seconds. Then, the gold-evaporated glass substrate was washed by shaking in water for 30 minutes, and dried.

On the other hand, a solution not containing any nucleic acid (1 × TE buffer) was also subjected to the immobilization operation as a control by spotting it on the gold-evaporated glass substrate in a similar manner.

### Comparative Example 7

Each of the λ DNA solutions (concentration: 1 pmol/µl) described in Example 7 was spotted on the gold-evaporated glass substrate as three spots at predetermined positions by using a spotter (Pyxsis 5500, CARTESIAN). This plate was put into a drier and dried at 42°C for 20 minutes. Then, the gold-evaporated glass substrate was washed by shaking in water for 30 minutes, and dried.

### Example 14: Hybridization and detection thereof

The λ DNA-immobilized gold-evaporated glass substrate of each of Example 13 and Comparative Example 7 was immersed in water heated to 95°C for 10 minutes, and immersed in water cooled to 4°C for 5 minutes. Subsequently, on the nucleic acid-immobilized portions of the λ DNA-immobilized gold-evaporated glass substrate, 60 ml of a hybridization solution (Arrayit UniHyb, (TeleCHem International, Inc.) containing 1 pmol of the biotinylated λ DNA (C) described in Example 8 was placed, and the gold-evaporated glass substrate was put into a case shielded from water (HybriCassette), immersed in a water bath with the case, and heated at 60°C for 2 hours.

Thereafter, post-hybridization and detection of hybridization were performed in the same manner as that of Example 8. The results are shown in Table 7.

**Table 7**

| | Immobilized nucleic acid | |
|---|---|---|
| | λ DNA fragment (A) | λ DNA fragment (B) |
| Example 13 | ⓞ | × |
| Comparative Example 7 | × | × |

As apparent from the results shown in Table 7, the λ DNA fragments were surely immobilized on the λ DNA-immobilized gold-evaporated glass substrate of Example 13, because the hybridization signals specifically and clearly appeared on the gold-evaporated glass substrate. On the other hand, no signal appeared at all on the λ DNA-immobilized gold-evaporated glass substrate of Comparative Example 7. In addition, also on the positions of control (positions at which a solution not containing any nucleic acid was spotted) of the λ DNA-immobilized gold-evaporated glass substrate of Example 13 and the λ DNA-immobilized gold-evaporated glass substrate of Comparative Example 7, no signal appeared at all.

### Example 15: Immobilization of nucleic acid on plate

Each of the λ DNA solutions described in Example 7 was spotted on a commercially available copper foil (Nikko Metal Manufacturing Co., Ltd) as three spots at predetermined positions by using a spotter (Pyxsis 5500, CARTESIAN). The size of each of the spots was about 0.3 mm in diameter. This copper foil was put into a drier and dried at 42°C for 20 minutes. Then, the foil was irradiated with an ultraviolet ray containing a component having a wavelength of 254 nm for 1,200 mJ/cm² by using Uvstratalinker 2400 (STRATAGENE) at a distance of 16 cm. The irradiation time was 480 seconds. Then, the copper foil was washed by shaking in water for 30 minutes, and dried.

On the other hand, a solution not containing any nucleic acid (1 × TE buffer) was also subjected to the immobilization operation as a control by spotting it on the copper foil in a similar manner.

### Comparative Example 8

Each of the λ DNA solutions (concentration: 1 pmol/µl) described in Example 7 was spotted on the copper foil as three spots at predetermined positions by using a spotter (Pyxsis 5500, CARTESIAN). This copper foil was put into a drier and dried at 42°C for 20 minutes. Then, the copper foil was washed by shaking in water for 30 minutes, and dried.

### Example 16: Hybridization and detection thereof

The λ DNA-immobilized copper foil of each of Example 15 and Comparative Example 8 was immersed in water heated to 95°C for 10 minutes, and immersed in water cooled to 4°C for 5 minutes. Subsequently, on the nucleic acid-immobilized portions of the λ DNA-immobilized copper foil, 60 ml of a hybridization solution (Arrayit UniHyb, (TeleCHem International, Inc.) containing 1 pmol of the biotinylated λ DNA (C) described in Example 8 was placed, and the copper foil was put into a case shielded from water (HybriCassette), immersed in a water bath with the case, and heated at 60°C for 2 hours.

Thereafter, post-hybridization and detection of hybridization were performed in the same manner as that of Example 8. The results are shown in Table 8.

**Table 8**

| | Immobilized nucleic acid | |
|---|---|---|
| | λ DNA fragment (A) | λ DNA fragment (B) |
| Example 15 | ⓞ | × |
| Comparative Example 8 | × | × |

As apparent from the results shown in Table 8, the λ DNA fragments were surely immobilized on the λ DNA-immobilized copper foil of Example 15, because the hybridization signals specifically and clearly appeared on the copper foil. On the other hand, no signal appeared at all on the λ DNA-immobilized copper foil of Comparative Example 8. In addition, also on the positions of control (positions at which a solution not containing any nucleic acid was spotted) of the λ DNA-immobilized gold-evaporated glass substrate of Example 15 and the λ DNA-immobilized copper foil of Comparative Example 8, no signal appeared at all.

### Example 17: Immobilization of nucleic acid on plate

Each of the λ DNA solutions described in Example 7 was spotted on a commercially available pure nickel foil (Nikko Metal Manufacturing Co., Ltd) as three spots at predetermined positions by using a spotter (Pyxsis 5500, CARTESIAN). The size of each of the spots was about 0.3 mm in diameter. This pure nickel foil was put into a drier and dried at 42°C for 20 minutes. Then, the foil was irradiated with an ultraviolet ray containing a component having a wavelength of 280 nm for 1,200 mJ/cm² by using Uvstratalinker 2400 (STRATAGENE) at a distance of 16 cm. The irradiation time was 480 seconds. Then, the pure nickel foil was washed by shaking in water for 30 minutes, and dried.

On the other hand, a solution not containing any nucleic acid (1 × TE buffer) was also subjected to the immobilization operation as a control by spotting it on the pure nickel foil in a similar manner.

### Comparative Example 9

Each of the λ DNA solutions (concentration: 1 pmol/µl) described in Example 7 was spotted on the pure nickel foil as three spots at predetermined positions by using a spotter (Pyxsis 5500, CARTESIAN). This pure nickel foil was put into a drier and dried at 42°C for 20 minutes. Then, the pure nickel foil was washed by shaking in water for 30 minutes, and dried.

### Example 18: Hybridization and detection thereof

The λ DNA-immobilized pure nickel foil of each of Example 17 and Comparative Example 9 was immersed in water heated to 95°C for 10 minutes, and immersed in water cooled to 4°C for 5 minutes. Subsequently, on the nucleic acid-immobilized portions of the λ DNA-immobilized pure nickel foil, 60 ml of a hybridization solution (Arrayit UniHyb, (TeleCHem International, Inc.) containing 1 pmol of the biotinylated λ DNA (C) described in Example 8 was placed, and the pure nickel foil was put into a case shielded from water (HybriCassette), immersed in a water bath with the case, and heated at 60°C for 2 hours.

Thereafter, post-hybridization and detection of hybridization were performed in the same manner as that of Example 8. The results are shown in Table 9.

**Table 9**

| | Immobilized nucleic acid | |
|---|---|---|
| | λ DNA fragment (A) | λ DNA fragment (B) |
| Example 17 | ⓞ | × |
| Comparative Example 9 | × | × |

As apparent from the results shown in Table 9, the λ DNA fragments were surely immobilized on the λ DNA-immobilized copper foil of Example 17, because the hybridization signals specifically and clearly appeared on the pure nickel foil. On the other hand, no signal appeared at all on the λ DNA-immobilized pure nickel foil of Comparative Example 9. In addition, also on the positions of control (positions at which a solution not containing any nucleic acid was spotted) of the λ DNA-immobilized pure nickel foil of Example 17 and the λ DNA-immobilized pure nickel foil of Comparative Example 9, no signal appeared at all.

### Example 19

Oligonucleotides having the nucleotide sequences of SEQ ID NOS: 9, 10, and 11 respectively (26mer) were synthesized in a conventional manner by using an oligonucleotide synthesizer (Perkin-elmer Applied Biosystems). The oligonucleotide having the nucleotide sequence of SEQ ID NO: 9 was biotinylated at the 5' end. The oligonucleotides having the nucleotide sequences of SEQ ID NOS: 9 and 10 corresponded to the oligonucleotides having the nucleotide sequences of SEQ ID NO: 1 and 2 described in Example 1 with five thymidine residues at the 5' ends, respectively. The oligonucleotide of SEQ ID NO: 11 did not have complementarity because it was different from the oligonucleotide of SEQ ID NO: 5 by one nucleotide. In other words, those oligonucleotides are the oligonucleotide sequences of SEQ ID NOS: 4, 5, and 6 as described in Example 3 with the number of the thymidine residues at the 5' end reduced to five.

The above oligonucleotides were immobilized in the same manner as in Example 3 using a commercially available stainless plate (Special Kinzoku Kogyo Co., Ltd.). Thereafter, post-hybridization, and detection of the oligonucleotides immobilized on the plates and hybridization were performed in the same manner as that of Example 2.

### Comparative Example 10

Each oligonucleotide was immobilized on a stainless plate in the same manner as in Comparative Example 2, except that oligonucleotide solutions described in Example 19 were used.

### Example 20

On the nucleic acid-immobilized portions of the oligonucleotide-immobilized plates of Example 19 and Comparative Example 10, 60 ml of a hybridization solution (Arrayit UniHyb, (TeleCHem International, Inc.) containing 3 pmol of the biotinylated probe (262bp) was placed, and the plates were put into a case shielded from water (HybriCassette), immersed in a water bath with the case, and heated at 45°C for 2 hours.

Thereafter, post-hybridization, and detection of the oligonucleotides immobilized on the plates and hybridization were performed in the same manner as that of Example 2. The results are shown in Table 10. The signals of the positions at which the oligonucleotide having the sequence of SEQ ID NO: 9 was immobilized indicate amounts of immobilized oligonucleotides, and the signals of the positions at which the oligonucleotide having the sequence of SEQ ID NO: 10 was immobilized indicate intensities of hybridization.

**Table 10**

| | Immobilized oligonucleotide | | |
|---|---|---|---|
| | SEQ ID NO: 9 | SEQ ID NO: 10 | SEQ ID NO: 11 |
| Example 19 | ⓞ | ⓞ | × |
| Comparative Example 10 | × | × | × |

### Industrial Applicability

According to the method of the present invention, a biomolecule, for example, a nucleic acid, especially a short chain length nucleic acid, can be conveniently and efficiently immobilized on a metallic carrier. Further, as coating of the carrier surface is unnecessary, a biomolecule may be directly fixed onto a metallic electrode or the like.

## Claims

1. A method of immobilizing a biomolecule on a carrier, comprising the steps of: spotting a solution of the biomolecule on the carrier; and irradiating the carrier spotted with the solution of the biomolecule with an ultraviolet ray containing a component having a wavelength of 280 nm, wherein the carrier is made of a metal.

2. The method according to claim 1, wherein the ultraviolet ray contains a component having a wavelength of 220 to 300 nm.

3. The method according to claim 1 or 2, wherein the metal is a metal selected from Groups I, II, III, IV, V, VI, and VII of second to seventh periods and transition elements in a periodic table, or an alloy containing any of these metal.

4. The method according to any one of claims 1 to 3, wherein irradiation dose of the ultraviolet ray is 100 mJ/cm² or more.

5. The method according to any one of claims 1 to 4, wherein the biomolecule is selected from a nucleic acid, protein, saccharide, antigen, antibody, peptide, and enzyme.

6. A method of producing a biomolecule-immobilized carrier in which a biomolecule is immobilized on a carrier, comprising the steps of: spotting a solution of the biomolecule on the carrier; and irradiating the carrier spotted with the solution of the biomolecule with an ultraviolet ray containing a component having a wavelength of 280 nm to immobilize the biomolecule on the carrier.

7. The method according to claim 6, wherein the ultraviolet ray contains a component having a wavelength of 220 to 300 nm.

8. The method according to claim 6, wherein the biomolecule comprises a nucleic acid, and the nucleic acid-immobilized carrier is used for analysis of the nucleic acid by hybridization.
